(19) Europäisches Patentamt
European Patent Office
Office européen des brevets

(11) **EP 3 695 879 A1**

(12) **EUROPEAN PATENT APPLICATION**

(43) Date of publication:
**19.08.2020 Bulletin 2020/34**

(51) Int Cl.:
***A61N 1/36*** (2006.01)

(21) Application number: **19156852.6**

(22) Date of filing: **13.02.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Ecole Polytechnique Federale De Lausanne (EPFL) EPFL-TTO
1015 Lausanne (CH)**
• **University Of Fribourg
1700 Fribourg (CH)**

(72) Inventors:
• ZHUANG, Katie
1007 Lausanne (CH)
• BARRA, Beatrice
1007 Lausanne (CH)
• LOSANO, Elena
51031 Agliana (IT)
• COURTINE, Grégoire
1005 Lausanne (CH)
• CAPOGROSSO, Marco
Pittsburg, PA 15206 (US)

(74) Representative: **DTS Patent- und Rechtsanwälte Schnekenbühl und Partner mbB
Marstallstrasse 8
80539 München (DE)**

(54) **A SYSTEM FOR PROVIDING NEUROMODULATION, ESPECIALLY NEUROSTIMULATION**

(57) A system (10) for providing neuromodulation, especially neurostimulation, comprising
- at least one neuromodulator (12) for neuromodulation of the cervical spinal cord,
- at least one dorsal neuromodulation array (14) for providing neuromodulation to the cervical spinal cord,
- at least one sensor (16) for measuring movements and/or muscle activations of a patient,
- at least one mapping module (18), which is configured and arranged for implementing a mapping between desired muscle activation patterns and neuromodulation parameters, and
at least one processing module (20) which is configured and arranged for processing signals provided by the at least one sensor (16), further forwarding the processed signals to the mapping module (18) and directing the mapping module output to the neuromodulator (12).

Fig. 1

**Description**

The present invention relates to a system for providing neuromodulation, especially neurostimulation.

The World Health Organization estimates that about half of a million people worldwide suffer a spinal cord injury (SCI) each year (World Health Organization & International Spinal Cord Society. International perspectives on spinal cord injury, World Health Organization (2013)).

Among the most disruptive impairments are the ones to the upper limbs, which result in deficiencies to three-dimensional reaching and grasping. At present, there is no effective therapy for this condition. An emerging field of research, epidural electrical stimulation (EES), has been shown to restore weight-bearing locomotion abilities and voluntary leg movements in both animal models and in humans with SCI. More interestingly, spatiotemporal EES bursts have proved to enable modulation and control of a broad spectrum of leg movements in rats and nonhuman primates (Wenger N, et al., Spatiotemporal neuromodulation therapies engaging muscle synergies improve motor control after spinal cord injury. Nat. Med. 22, 138 (2016); Wenger N, et a/., Closed-loop neuromodulation of spinal sensorimotor circuits controls refined locomotion after complete spinal cord injury. Sci. Trans/. Med. 6, 255ra133 (2014); Capogrosso M, et al., A brain-spine interface alleviating gait deficits after spinal cord injury in primates. Nature 539, 284 (2016)). While evidence of EES's effectiveness for upper limb movement recovery is still sparse, recent results show promise. Continuous EES protocols applied in patients with spinal cord injury result in improvements in voluntary grasping functions (Lu, DC. et al. Engaging Cervical Spinal Cord Networks to Reenable Volitional Control of Hand Function in Tetraplegic Patients. Neurorehabil. Neural Repair 30, 951-962 (2016)). Another recent study showed that cervical EES was able to consistently recruit specific muscles in anesthetized macaques and that these muscle responses were graded depending on the properties of the stimulation (Sharpe AN, et al., Upper-limb muscle responses to epidural, subdural and intraspinal stimulation of the cervical spinal cord. J. Neural Eng. 11, 016005-016005 (2014)).

Decades of research in physiology have demonstrated that the mammalian spinal cord embeds sensorimotor circuits that produce movement primitives (cf. Bizzi E, et al., Modular organization of motor behavior in the frog's spinal cord. Trends in neurosciences 18, 442-446 (1995); Levine AJ, et al., Identification of a cellular node for motor control pathways. Nature neuroscience 17, 586-593, (2014)). These circuits process sensory information arising from the moving limbs and descending inputs originating from various brain regions in order to produce adaptive motor behaviours.

A SCI interrupts the communication between the spinal cord and supraspinal centres, depriving these sensorimotor circuits from the excitatory and modulatory drives necessary to produce movement.

A series of studies in animal models and humans showed that electrical neuromodulation of the lumbar spinal cord using EES is capable of reactivating these circuits. For example, EES has restored coordinated locomotion in animal models of SCI, and isolated leg movements in individuals with motor paralysis (cf. van den Brand R, et al., Restoring Voluntary Control of Locomotion after Paralyzing Spinal Cord Injury. Science 336, 1182-1185 (2012); Angeli CA., et a/., Altering spinal cord excitability enables voluntary movements after chronic complete paralysis in humans. Brain: a journal of neurology 137, 1394-1409 (2014); Harkema S, et al., Effect of epidural stimulation of the lumbosacral spinal cord on voluntary movement, standing, and assisted stepping after motor complete paraplegia: a case study. The Lancet 377, 1938-1947; Danner SM, et al., Human spinal locomotor control is based on flexibly organized burst generators. Brain: a journal of neurology 138, 577-588 (2015); Courtine G, et al., Transformation of nonfunctional spinal circuits into functional states after the loss of brain input. Nature neuroscience 12, 1333-1342, (2009); Capogrosso M, et al., A brain-spine interface alleviating gait deficits after spinal cord injury in primates. Nature 539, 284-288, (2016)).

Computational models (cf. Capogrosso M, et al., A computational model for epidural electrical stimulation of spinal sensorimotor circuits. The Journal of neuroscience: the official journal of the Society for Neuroscience 33, 19326-19340 (2013); Moraud EM, et al., Mechanisms Underlying the Neuromodulation of Spinal Circuits for Correcting Gait and Balance Deficits after Spinal Cord Injury. Neuron 89, 814-828 (2016); Rattay F, et al., Epidural electrical stimulation of posterior structures of the human lumbosacral cord: 2. quantitative analysis by computer modeling. Spinal cord 38, 473-489 (2000)) and experimental studies (cf. Gerasimenko Y, et al., Program No. 447.445 (Soc. Neurosci. Abstr.); Minassian K, et al., Human lumbar cord circuitries can be activated by extrinsic tonic input to generate locomotor-like activity. Human Movement Science 26, 275-295 (2007)) have provided evidence suggesting that EES recruits large sensory afferents, especially proprioceptive circuits (cf. Moraud EM, et al., Mechanisms Underlying the Neuromodulation of Spinal Circuits for Correcting Gait and Balance Deficits after Spinal Cord Injury. Neuron 89, 814-828 (2016)).

Consequently, the stimulation leads to the activation of motoneurons through mono- and polysynaptic proprioceptive circuits, as well as upregulating the general excitability of the lumbar spinal cord. In addition, the natural modulation of proprioceptive circuits during movement execution gates the effects of EES towards functionally relevant pathways. Concretely, due to phase-dependent modulation of proprioceptive circuits, the effects of stimulation are restricted to specific ensembles of leg motoneurons that are coherent with the phase of the movement (cf. Moraud EM, et al. Mechanisms Underlying the Neuromodulation of Spinal Circuits for Correcting Gait and Balance Deficits after Spinal Cord Injury. Neuron 89, 814-828 (2016)).

Moreover, since EES engages motoneurons through pre-synaptic mechanisms, residual inputs from supraspinal centres are also capable of gating the effects of EES towards specific circuits in order to mediate voluntary modulation of leg movements *(cf.* van den Brand R, et al., Restoring Voluntary Control of Locomotion after Paralyzing Spinal Cord Injury. Science 336, 1182-1185 (2012*); Angeli CA, et al., Altering spinal cord excitability enables voluntary movements after chronic complete paralysis in humans. Brain: a journal of neurology 137, 1394-1409 (2014); Harkema S. et al., Effect of epidural stimulation of the lumbosacral spinal cord on voluntary movement, standing, and assisted stepping after motor complete paraplegia: a case study. The Lancet 377 (1938-1947))*.

This conceptual framework was exploited to design a neuromodulation strategy that targets specific ensembles of proprioceptive afferents associated with flexion and extension of both legs (cf. Bizzi E, et al, Modular organization of motor behavior in the frog's spinal cord. Trends in neurosciences 18, 442-446 (1995*); Levine AJ, et al. Identification of a cellular node for motor control pathways. Nature neuroscience 17, 586-593 (2014))*.

This strategy, termed spatiotemporal neuromodulation, consists of delivering EES bursts through targeted electrode configurations with a temporal structure that reproduces the natural activation of leg motoneurons during locomotion. This spatiotemporal neuromodulation therapy reversed leg paralysis in both rodent and primate models of SCI *(cf.* Capogrosso M, et al., A brain-spine interface alleviating gait deficits after spinal cord injury in primates. Nature 539, 284-288 (2016*); Wenger N, et al., Spatiotemporal neuromodulation therapies engaging muscle synergies improve motor control after spinal cord injury. Nat Med 22, 138-145 (2016))*.

This conceptual framework is applicable to develop spatiotemporal neuromodulation therapies for enabling leg motor control in humans with SCI.

Generally speaking, known stimulation systems use either Central Nervous System (CNS) stimulation, especially EES, or Peripheral Nervous System (PNS) stimulation, especially Functional Electrical Stimulation (FES).

EES is known to restore motor control in animal and human models and has more particularly been shown to restore locomotion after spinal cord injury by artificially activating the neural networks responsible for locomotion below the spinal cord lesion (cf. Capogrosso M, et al., A Computational Model for Epidural Electrical Stimulation of Spinal Sensorimotor Circuits, Journal of Neuroscience 4 December 2013, 33 (49) 19326-19340*; Courtine G, et al., Transformation of nonfunctional spinal circuits into functional states after the loss of brain input, Nat Neurosci. 12(10): 1333-1342 (2009); Moraud EM, et al., Mechanisms Underlying the Neuromodulation of Spinal Circuits for Correcting Gait and Balance Deficits after Spinal Cord Injury, Neuron Volume 89, Issue 4, p814-828 (2016))*. EES does not directly stimulate motor-neurons but the afferent sensory neurons prior to entering into the spinal *cord.* In this way, the spinal networks responsible for locomotion are recruited indirectly via those afferents, restoring globally the locomotion movement by activating the required muscle synergies. The produced movement is functional.

PNS stimulation systems used to date in the clinic employ FES and provide electrical stimulation to target muscles with surface electrodes, either directly through stimulation of their motor fibers (neuro-muscular stimulation), through a limited set of reflexes (practically limited to the withdrawal reflex), or by transcutaneously stimulating the peripheral nerves. The resulting muscle fatigue has rendered FES unsuitable for use in daily life. Furthermore, successes have remained limited through cumbersome setups when using surface muscle stimulation, unmet needs in terms of selectivity (when using transcutaneous nerve stimulation) and a lack of stability (impossible to reproduce exact electrode placement on a daily basis when stimulating muscles, electrode migration due to clothing, sweating).

US 2016/030750 A1 discloses a computer implemented system and method that facilitates the generation, sharing and refinement of volumes to stimulate anatomical tissue, such as spina! cord stimulation. The computer system analyses the volumes as well. More specifically, a computer implemented system and method facilitates a cycle of generation, sharing, and refinement of volumes related to stimulation of anatomical tissue, such as brain or spinal cord stimulation. Such volumes can include target stimulation volumes, side effect volumes, and volumes of estimated activation. A computer system and method also facilitate analysis of groups of volumes, including analysis of differences and/or commonalities between different groups of volumes.

US 2016/001096 A1 describes methods and systems that use multiple therapeutic modalities to cause deep or superficial deep-brain stimulation. Methods for treatment of clinical conditions and physiological impacts are described, as well as methods for Guided Feedback control of non-invasive deep brain or superficial neuromodulator, as well as the non-invasive neuromodulation of the spinal cord by ultrasound energy.

EP 2 810 689 A1 and EP 2 810 690 A1 describe a system for planning and providing a therapy for Deep Brain neural applications, especially neurostimulation and/or neurorecording with at least one lead with a plurality of electrodes. The invention concerns a method for focusing the stimulation field provided by an active contact of a lead.

US 2015/066111 A1 discloses a tool for assisting in the planning or performing of electrical neuromodulation of a patient's spinal cord by calculating a volume of activation, registering electrodes and their position.

Current systems for neuromodulation in the field of the treatment of spinal cord injuries (SCI), for example after trauma or stroke or illness, must match each input signal to a specific reaction of the patient. This can be quite time-consuming and also exhaustive for the patient, physician and medical support staff.

It is an object of the present invention to improve a system for planning and/or providing neuromodulation, especially in connection with the treatment of spinal cord injuries (SCI), for example after trauma or stroke or illness, especially in that the procedure of linking input signals and the neuromodulation provided with the desired reaction of the patient is simplified and less time-consuming.

This object is solved according to the present invention by providing a system for neuromodulation according to claim 1. Accordingly, there is provided a system for providing neuromodulation, especially neurostimulation, comprising

- at least one neuromodulator for neuromodulation of the cervical spinal cord,
- at least one dorsal neuromodulation array for providing neuromodulation to the cervical spinal cord,
- at least one sensor for measuring movements and/or muscle activations of a patient,
- at least one mapping module, which is configured and arranged for implementing a mapping between desired muscle activation patterns and neuromodulation parameters, and
- at least one processing module which is configured and arranged for processing signals provided by the at least one sensor, further forwarding the processed signals to the mapping module and directing the mapping module output to the neuromodulator.

The invention is based on the basic idea that a system for providing neuromodulation is provided, especially for patients with paralysis and/or paraplegia. In particular, the system is aiming for a solution in which recovery of reaching and grasping movements can be provided. The basic idea further includes the step of characterizing the muscle activity patterns of e.g. healthy persons movement tasks, e.g. during three-dimensional reaching and grasping tasks. In particular, the measured movements and/or muscle activations of a patient by means of the sensor(s) are used, to implement a mapping between desired muscle activation patterns and neuromodulation parameters. This implementation step is done by means of the mapping module and in connection with the processing module. The processing module processes the signals provided by the at least one sensor, further forwards the processed signals to the mapping module and directs the mapping module output to the neuromodulator. Thus, it is possible to be selective for muscle activations rather than motoneurons in the spinal segments. In particular, the system is designed for neuromodulation of the cervical spinal cord and for restoration of movement capability of the upper limbs and/or upper body parts of the human body, but not limited to that. In the cervical spinal cord, we are able to achieve far greater stimulation specificity than in the lumbar spinal cord due to the more spatially separated posterior roots of the cervical spinal *cord.* Thus, the system is able to target distinct muscle groups more specifically when compared with existing systems. Generally speaking it is of course possible to combine the system according to the present invention and/or one of its embodiments with one or more existing systems, e.g. a system to deliver adaptive electrical spinal cord stimulation to facilitate and restore locomotion after a neuromotor impairment (EP 2 868 343 A1), a pulse generating system (EP 3 269 424 A1), a system for neuromodulation (EP 3 421 081 A1), a neurostimulation system for central nervous stimulation (CNS) and peripheral nervous stimulation (PNS) (EP 3 381 506 A1).

The system is for example able to perform a novel stimulation algorithm for choosing stimulation parameters in real time. The algorithm depends on stimulation at one or multiple sites in order to achieve any desired muscle activation map. However, the choice of the sites is limited such that stimulation sites with overlapping muscle activations can be chosen simultaneously. Within this algorithm a metric for assessing this activation overlap and use it to automate stimulation site selection is defined.

It is further possible that the neuromodulator is a neurostimulator. In particular, the neurostimulator can be an EES stimulator.

For EES, an electrode array may be positioned within the epidural space (i.e. on the outside of the dural sac, which encases the spinal cord and the cerebrospinal fluid in which the spinal cord 'floats'), on top of the spinal cord.

The stimulation parameters for EES stimulation may be frequency, amplitude, pulse-width and the like.

Moreover, it is possible that the neurostimulator is configured and arranged to provide neurostimulation in at least three channels independently. By providing at least three channels, tailor-made and adjusted stimulation for complex movements can be provided. In other words, by providing three or more stimulation channels the system is capable to assist such complex movements like grasping or similar three-dimensional movements more precise and thus allows better stimulation outcome.

Preferably, the neurostimulator may be configured and arranged to provide neurostimulation in a frequency range from 0-1000Hz, preferably between 10-1000Hz. This range has been identified in various tests as a range, which allows and provides good stimulation results.

The neuromodulator may be at least partially implanted, especially fully implanted. An implanted neuromodulator allows a more precise and targeted neuromodulation of the posterior roots and is therefore preferred.

The dorsal neuromodulation array may be a dorsal neurostimulation array. In particular, the dorsal neurostimulation array may be configured and arranged for an implantation in the region of the cervical spinal cord. Further, it can be configured and arranged for providing EES stimulation that targets the posterior roots.

The neuromodulation array may have a plurality of active sites, wherein the active sites are placed in a region of approx. 2-25 mm, especially approx. 4-10 mm from the midline of the patient's spinal cord.

Generally speaking, there is no a set range, but the mentioned ranges have proven to be very effective and advantageous. Generally, the dimensions should be chosen such they depend on the spacing of the patient's spinal cord.

The neuromodulation may be a neuromodulation for selective muscle activation.

In general, the neuromodulation may target posterior roots.

The mapping module may be embodied as a microcontroller or as a computer.

Also, the processing module may be embodied as a microcontroller or as a computer

In particular, the processing module may be embodied as an implantable processor.

Especially, the mapping module and the processing module may be embodied in the same microcontroller or computer.

Further details and advantages of the present invention shall now be described in greater detail in connection with the drawings.

It is shown in

Fig. 1    a schematical overview of an embodiment of the system according to the present invention, with which the method according to the present invention can be performed;

Fig. 2    a muscle activation map during the natural performance of the task, performed with the embodiment of the system and method according to the present invention according to Fig. 1;

Fig. 3    recruitment curves and selectivity indices used in the embodiment of the system and method according to the present invention according to Fig. 1; and

Fig. 4    a task specific reconstruction of spinal segments spatiotemporal activation map.

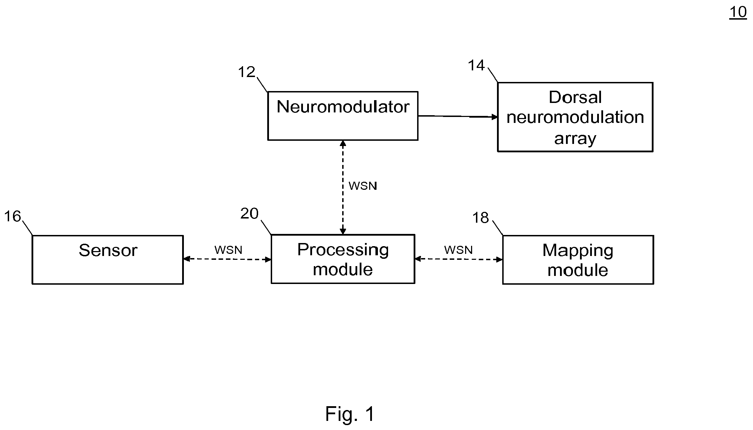

**Fig. 1** shows a schematical overview of an embodiment of the system 10 according to the present invention, with which the method according to the present invention can be performed.

The system 10 comprises a neuromodulator 12.

Alternatively, the system 10 could comprise more than one neuromodulator 12.

Further, the system 10 comprises a dorsal neuromodulation array 14.

Alternatively, the system 10 could comprise more than one dorsal neuromodulation arrays 14.

The system 10 further comprises a sensor 16.

In an alternative embodiment the system 10 could comprises more than one sensor 16.

The system 10 further comprises a mapping module 18.

Alternatively, the system 10 could comprise more than one mapping module 18.

The system 10 further comprises a processing module 20.

The neuromodulator 12 is connected to the dorsal neuromodulation array 14.

The sensor 16 is connected to the processing module 20.

The processing module 20 is connected to the mapping module 18.

The processing module 20 is also connected to the neuromodulator 12.

The connection between the neuromodulator 12 and the dorsal neuromodulation array 14 is in the shown embodiment a direct and unidirectional connection.

However, also an indirect (i.e. with another component of the system 10 in between) and/or bidirectional connection would be generally possible.

The connection between the neuromodulator 12 and the dorsal neuromodulation array 14 is in the shown embodiment a cable-bound connection.

However, also a wireless connection, e.g. by a wireless network WSN, could be generally possible.

The connection between the sensor 16 and the processing module 20 is in the shown embodiment a direct and bidirectional connection.

However, also an indirect (i.e. with another component of the system 10 in between) and/or unidirectional connection would be generally possible.

The connection between the sensor 16 and the processing module 20 is in the shown embodiment established by a wireless network WSN.

However, also a cable-bound connection could be generally possible.

The connection between the processing module 20 and the mapping module 18 is in the shown embodiment a direct and bidirectional connection.

However, also an indirect (i.e. with another component of the system 10 in between) and/or unidirectional connection would be generally possible.

The connection between the processing module 20 and the mapping module 18 is in the shown embodiment established by a wireless network WSN.

However, also a cable-bound connection could be generally possible.

The connection between the processing module 20 and the neuromodulator 12 is in the shown embodiment a direct and bidirectional connection.

However, also an indirect (i.e. with another component of the system 10 in between) and/or unidirectional connection would be generally possible.

The connection between the processing module 20 and the neuromodulator 12 is in the shown embodiment established by a wireless network WSN.

However, also a cable-bound connection could be generally possible.

The neuromodulator 12 provides neuromodulation to the cervical spinal cord of a patient.

In particular, the neuromodulation is provided by a dorsal neuromodulation array 14 to the cervical spinal cord of a patient.

In this embodiment, the neuromodulation is a neuromodulation for selective muscle activation.

In this embodiment, the neuromodulator 12 is a neurostimulator 12 and provides neurostimulation to the cervical spinal cord of a patient via the dorsal neuromodulation array 14, which is a dorsal neurostimulation array.

In particular, the neurostimulator 12 provides neurostimulation to posterior roots of a patient.

In general, the neuromodulator 12 could be partially implanted in the body of a patient.

However, the neuromodulator 12 also could be fully implanted in the body of a patient.

In particular, the neuromodulator 12 provides neuromodulation in a frequency range from 0-1 000 Hz, preferably between 10 and 1000 Hz.

A response to the provided neuromodulation is measured by the sensor 16.

In this embodiment, the response to the provided neuromodulation is/are muscle activations of a patient.

In an alternative embodiment, the response to the provided neuromodulation are additionally and/or alternatively movements of a patient.

In general, every other type of physiological response to the neuromodulation could be measured by the sensor 16.

The mapping module 18 implements a mapping between desired muscle activation patterns and neuromodulation parameters.

The processing module 20 processes signals provided by the sensor 16, forwards the processed signals to the mapping module 18 and directs the mapping module output to the neuromodulator 12.

Not shown in Fig. 1 is that the neurostimulator 12 provides neurostimulation in at least three channels independently and simultaneously.

However, also fewer than three channels may be chosen at any given time.

Not shown in Fig. 1 is that the dorsal neuromodulation array 14 could have a plurality of active sites E, wherein the active sites E are placed in a region of approx. 2-25 mm, especially 4-10 mm from the midline of the patient's spinal cord.

Not shown in Fig. 1 is that at least one sensor 16 could measure brain signals.

Not shown in Fig. 1 is that brain signals could be measured by extracellular recording.

Not shown in Fig. 1 is that extracellular recording of brain signals may include single-unit recording, multi-unit recording, field potential recording and/or amperometry.

In general, not shown in Fig. 1 is that the at least one sensor 16 could be or could comprise an inertial measurement unit (IMU), an optical sensor, a camera, a piezo element, a velocity sensor, an accelerometer, a magnet sensor, a torque sensor, a pressure sensor, a force sensor, a displacement sensor, a contact sensor, an EEG measurement unit, an EMG measurement unit, a goniometer, a magnetic field sensor, a hall sensor and/or a gyroscope and/or a motion tracking video camera, or a infra-red camera.

Not shown in Fig. 1 is that two or more sensors 16 could form a sensor network 16.

Further not shown in Fig. 1 is that the mapping stage and the integration stage may be encompassed by the same computer/microcontroller and that the one or both are external to the patient partially implanted or fully implanted.

Not shown in Fig. 1 is that the sensor 16 could also be directly connected to the mapping module 18 and/or the neuromodulator 12 and/or the dorsal neuromodulation array.

Also not shown in Fig. 1 is that the dorsal neuromodulation array 14 could be directly connected to the mapping module 18 and/or the processing module 20.

Not shown in Fig. 1 is that the mapping module 18 may be embodied as a microcontroller or as a computer.

Not shown in Fig. 1 is that the processing module 20 may be embodied as a microcontroller or as a computer.

Further not shown in Fig. 1 is that the processing module 20 may be embodied as an implantable processor.

These connections could be unidirectional and/or bidirectional connections, wireless connections and/or cable-bound connections.

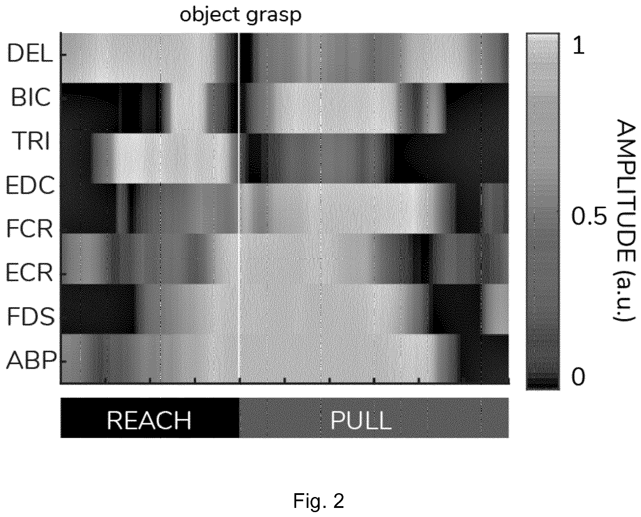

As shown in Fig. 2, Fig. 3, and Fig. 4 the feasibility of the above paradigm has been demonstrated in an animal model.

As already mentioned above, the system and the respective method according to the invention is an epidural stimulation control system for recovery of reaching and grasping movements after paralysis.

This is accomplished by first characterizing the muscle activity patterns in healthy subjects, e.g. as done by the inventors in two Macaca Fascicularis during three-dimensional reaching and grasping tasks. Then the stimulation specificity that can be achieved using cervical EES applied by a customized multi-site electrode is described. The stimulation parameters are selected automatically to achieve a desired muscle activation pattern. Finally, it is shown that the present invention can elicit the same patterns of muscle activation as observed in natural arm movements using EES delivered from only a few stimulation sites. The system elicits the same activation patterns of arm muscles as observed during natural three-dimensional reaching by utilizing the one-to-all connectivity between la proprioceptive afferents and motoneurons. The mechanism recruits these la afferents in the cervical roots, which monosynaptically and specifically recruit motoneurons.

SYSTEM DESCRIPTION

The system 10 is composed by the following components:

1. A fully implantable neuromodulator 12, i.e. a neurostimulator 12, able to stimulate at least 3 channels independently and simultaneously at different frequencies ranging from 10Hz to 1000Hz.

2. A dorsal neuromodulation array 14, i.e. a dorsal epidural array 14 with lateralized active sites to target the posterior roots of the cervical spinal cord with active sites placed anywhere between 4-10 mm from the midline.

3. A sensor 16 system measuring arm kinematics, muscle activity or brain signals.

4. A microcontroller that implements a mapping between desired muscle activation patterns and stimulation parameters.

5. An implantable processor that integrates the signals from the sensor 16 system, feeds these signals to the microcontroller and directs the microcontroller output to the implantable neurostimulator 12 which in turn delivers stimulation of the posterior roots via the dorsal epidural array 14.

INNOVATION STEP

The main innovation steps described here are described as follows:

1. Unlike previous approaches in connection with EES for motor recovery of the lower limbs, the goal of the present invention is to be selective for muscle activations rather than motoneurons in the spinal segments. In the cervical spinal cord, now it is possible to achieve far greater stimulation specificity than in the lumbar spinal cord due to the more spatially separated posterior roots of the cervical spinal cord. Thus, the algorithm for the system and method according to the present invention can be designed to target distinct muscle groups, which was not possible for the lumbar EES system. In particular, the neuromodulation is unlike in previous approaches a dorsal neuromodulation, which targets posterior roots.

2. A novel stimulation algorithm is described for choosing stimulation parameters in real time. The algorithm depends on stimulation at one or multiple sites in order to achieve any desired muscle activation map. However, the choice of the sites is limited such that stimulation sites with overlapping muscle activations should not be chosen simultaneously. It is defined a metric for assessing this activation overlap and use it to automate stimulation site selection.

METHODS

To design the stimulation protocol, the inventors conducted two experiments. First, they studied the three-dimensional reaching and grasping behavior of intact macaques to verify that a consistent spatiotemporal pattern of motor unit activation existed (Experiments 1A and 1B). Second, they tested our ability to use EES applied at the cervical level to selectively recruit groups of functionally activated muscles (Experiment 2). Using the results from all experiments, they developed a novel stimulation protocol to activate the motor units observed in natural reaching and grasping. Then, the stimulation results were compared with those of recorded muscle activity.

Animals and Behavioral Experiments

The experimental protocol was approved by the cantonal (Fribourg) and federal (Swiss) veterinary authorities (authorization No. 2014_42E_FR). Experiments were performed on two female macaque monkeys (Mk-CA and Mk-SA Macaca Fascicularis). Mk-CA performed a reach and grasp drawer task in Experiment 1A (Schmidlin E, et a/., Behavioral assessment of manual dexterity in non-human primates. J. Vis. Exp. JoVE 3258 (2011); Chatagny P, et al., Distinction between hand dominance and hand preference in primates: A behavioral investigation of manual dexterity in nonhuman primates (macaques) and human subjects. 3 (2013)) while Mk- SA performed, a three-dimensional reaching task in Experiment 1B. The Experiment 1A task consisted of reaching and opening a drawer, which contained a food reward. Mk-CA was trained to rest the hand on a pad while waiting for a starting cue. Once ready, the subject reached with the left arm to the drawer knob, opened the drawer and extracted the reward (food pellet) contained inside. The Experiment 1B task required Mk-SA to reach, grasp, pull and release a sensorized object presented by a robotic arm (KUKA LBR iiwa, KUKA CEE GmbH). In both monkeys, intramuscular electromyographic (EMG) activity from n=8 muscles was recorded during task execution: deltoid (DEL), biceps (BIC), triceps (TRI), extensor digitorum communis (EDC), wrist extensor (WRE), flexor digitorum superficialis (FDS), wrist flexor (WRF), abductor pollicis brevis (APB)).

For behavioral experiments, three movement phases were automatically extracted: arm extension, grasping and pulling, and movement end. The arm extension phase was defined to be from movement onset to target (drawer knob or sensorized object) acquisition. Pulling lasted from target grasp until either complete opening of drawer (Exp. 1A) or until release of the sensorized object (Exp. 1B). Movement end was defined by the picking of the reward in Experiment 1A or release of the sensorized object in Experiment 1B. Then projected the recorded EMG activity was projected to the anatomical location of the corresponding motoneurons in the spinal segments based on published studies (Jenny A, et al., Principles of motor organization of the monkey cervical spinal cord. J. Neurosci. 3, 567 (1983)) in order to reconstruct the spatiotemporal activation patterns of arm and hand motoneurons during each of these phases.

Recruitment curve experiments

Prior to Experiment 2, both monkeys underwent a surgical procedure in which a customized spinal implant, based on previously reported technology (Minev IR, et al., Electronic dura mater for long-term multimodal neural interfaces. Science 347, 159 (2015)), was surgically inserted into the epidural space of the cervical spinal cord. To gain access to the site, a laminectomy was executed at the vertebral level T1. Each spinal implant comprised n=7 independent electrodes E (which are here E1, E2, E3, E4, E5, E6, E8), or "active sites" through which current could be applied, and one "inactive" site E7. During Experiment 2, single pulse stimulation at different current amplitudes was delivered from each electrode of the spinal implant while intramuscular EMG activity was recorded from all 8 muscles for both monkeys. Then, the level of muscle recruitment over the stimulation current amplitudes for each active site were calculated separately and the recruitment curves were computed.

RESULTS

1. Identification of spinal cord activation patterns during movement (Experiment 1A and 1B)

The spatiotemporal maps of arm and hand muscle activation during reaching and grasping movements revealed a well-defined pattern of activation over movement phases (cf. Fig. 2, muscle activation map during the performance of a task). During the reach phase, the most active muscles are those of the deltoid and triceps muscles. These are used to stabilize and extend the arm towards the target. The transition to the grasp and pull phase was associated with the activation of extensor hand muscles together with flexor muscles of the forearm. An activation of the deltoid muscle then ensued for the rest of the pull phase. This activation pattern presented a series of well separated sequential muscle activation hotspots during the task. It was concluded that the reach and grasp tasks in Experiments 1A and 1B could be represented by a well-defined temporal pattern of muscle recruitment.

2. Muscle responses induced by cervical EES

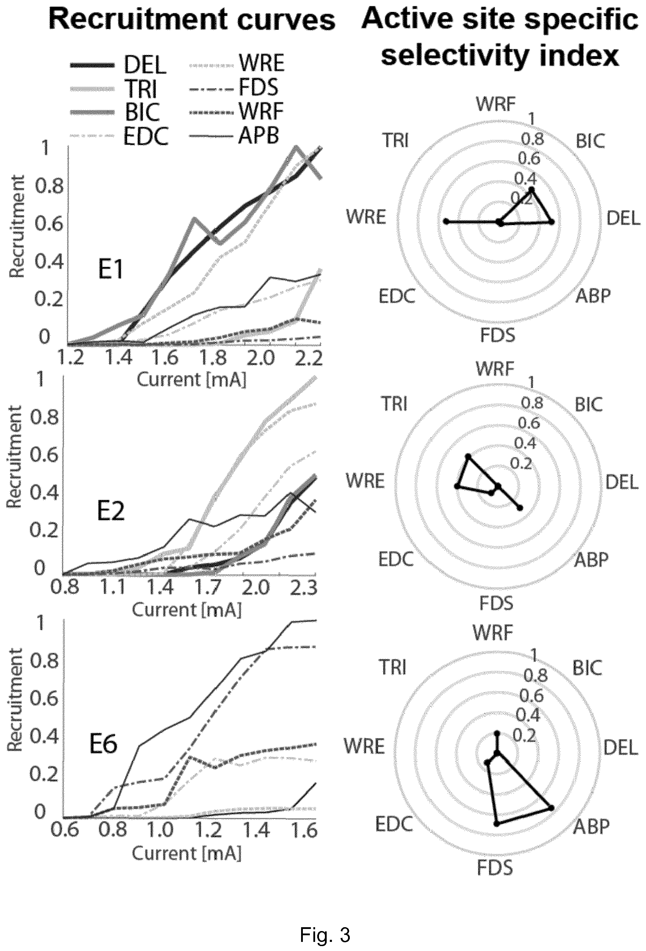

The recruitment curves computed for the different active sites highlighted a good spatial selectivity of the epidural implant, cf. **Fig. 3** showing muscle recruitment curves and selectivity indices of three epidural electrode active sites E1, E2 and E6 (from top to bottom) for Mk-Ca and Mk-Sa. These active sites enabled the selective activation of arm/hand flexors, arm/hand extensors and digit muscles, respectively. However, to elicit these activations, the active sites of the epidural implant were placed directly over the dorsal columns of the cervical spinal cord. This means that the width of the clinical electrode implant must span the width of the dorsal horns of the spinal cord (on average ∼ 7mm from midline) (Sherman JL, et al., Measurements of the normal cervical spinal cord on MR imaging. Am. J. Neuroradiol.

11, 369-372 (1990); Kirazli Ö, et al., Anatomy of the spinal dorsal root entry zone: its clinical significance. Acta Neurochir. (Wien) 156, 2351-2358 (2014)). It was concluded that EES of the cervical spinal cord may be able to access specific groups of functionally activated muscles.

## DESIGN OF STIMULATION CONTROL SYSTEM

Based on the results of our first experiment, it was found that it is possible to characterize the spatiotemporal motoneuron activations during reaching and grasping movements. These activation patterns are not fixed during movement execution, suggesting that EES stimulation parameters will need to be adjusted in real time throughout the movement duration. This hypothesis was supported by a pilot experiment in one monkey (Mk-Sa) in which continuous stimulation was delivered. This stimulation resulted in movement perturbation and inaccurate reaching. Thus, the proposed stimulation protocol was implemented in two steps: the calibration step and the online stimulation selection step.

The calibration step requires the identification of a mapping between stimulation active sites and the muscles activated for varying stimulation amplitudes. Thus, it is important to define accurate recruitment curves to construct this map. This was accomplished using the results obtained in Experiment 2 in which the optimal stimulation pins to evoke specific muscles were deduced.

In clinical application, single pulse EES at different amplitudes would be delivered during electrode implantation from each active site to map motoneuronal activations.

Thus the following algorithm and method can be performed by means of the system 10:
During movement execution, the intended muscular activation $\mu^d$ is continuously decoded from cortical signals or other biosignals.

Then, based on the recruitment curve mapping, the active site e and its relative amplitude $a_i^e$ are chosen in order to produce a motoneuronal activation $\mu(e, a_i^e)$ that would fit at best the decoded activation $\mu^d$:

$$\text{Equation 1 } e, a_i^e \ s.t. \min_{e, a_i^e} (\mu^d - \mu(e, a_i^e))$$

In order to account for coordinated multi-muscular activations, the selection of two concurrent active sites $e^1$ and $e^2$ is allowed under the following conditions:

$$\text{Equation 2. } \arccos\left(\frac{\mu(e^1, a_i^{e^1}) \cdot \mu(e^2, a_j^{e^2})}{\|\mu(e^1, a_i^{e^1})\| \cdot \|\mu(e^2, a_j^{e^2})\|}\right) > N^\circ$$

$$\text{Equation 3. } \left(\mu^d - \mu\left(e^1 + e^2, a_i^{e^1} + a_j^{e^2}\right)\right) < \left(\mu^d - \mu\left(e^3, a_k^{e^3}\right)\right), \forall e^3, a_k^{e^3}$$

The condition described in Eq. 2 prevents that two active sites eliciting responses from the same muscles would be concurrently activated up to a predefined threshold of N degrees, which can be set for each individual; Eq. 3 guarantees that the selection of a single electrode is always preferred over a combination of electrodes to reduce stimulation complexity.

These criteria can be scaled to as many active sites as desired (i.e. more than two active sites may be selected).

To implement the stimulation algorithm of the system 10, the implantable pulse generator (IPG) used must be able to deliver stimulation on at least three sites simultaneously.

In addition, the algorithm performs the updates to stimulation parameters at a rate of 10-100Hz, though it can function also outside of these frequency ranges, depending on the complexity of the movement.

Thus, the IPG must also be able to switch active sites and update all stimulation parameters (i.e. amplitude, frequency, pulse with, etc.) with a rate of at least 10Hz.

From Experiments 1A and 1B, it is possible to deduce the time-varying map of muscle activations during natural reaching and pulling.

The goal during task performance is to recreate these same activation sequences using EES.

From the activation map, a template set of muscle activations during the reach and pull phases was constructed to guide the stimulation parameter selection. To translate to a clinical setting, this template would be constructed using the unaffected arm or from averaged activities across a population of users and fine-tuned to the particular user before stimulator usage.

During real-time usage, reach and pull phases will be detected using either brain signals (by electroencephalography (EEG)) or other muscle/movement signals (by electromyography (EMG) of unaffected areas/inertial measurement units or other sensors).

This will then be passed to the implantable processor to finally activate the template electrode contacts to stimulate the correct muscles for the appropriate movement phase.

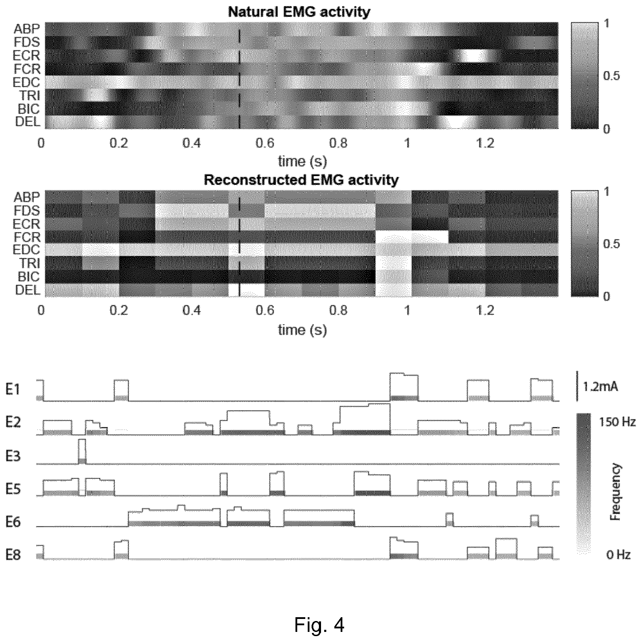

**Fig. 4** shows a task specific reconstruction of muscle envelope spatiotemporal activation map.

Top: Task specific spatiotemporal activation map obtained from muscle activation recording during behavioral experiments.

Middle: The task-specific spatiotemporal activation was reproduced selecting the muscle activation obtained from single pulse EES delivered from the electrode active sites.

Bottom: The active sites used (here, E1, E2, E3, E5, E6, E8), and their current amplitudes were chosen as the ones mimicking at best the activation pattern obtained during task performance. E4 was not used due to its lack of effective muscle recruitment.

Thus, **Fig. 4** illustrates an example of the performance of the described stimulation approach, where the muscle activation recorded during the performance of our 3D reach and grasp task is used as a proxy for the decoded motoneuronal activation $\mu^d$. In the stimulation selection step the implantable processor chooses the optimal stimulation parameters to elicit desired movement patterns. The resulting map showed a succession of spatially defined hotspots that were qualitatively similar to those found in the original task-specific map.

Note that the example control and estimation routines included herein can be used with various neuromodulation and/or neurostimulation system configurations. The control methods and routines disclosed herein may be stored as executable instructions in non-transitory memory and may be carried out by a control unit such as a microcontroller (or a computer) in combination with the at least one neuromodulator 12, dorsal neuromodulation array 14, sensor 16, mapping module 18, processing module 20 and/or other system 10 hardware. The specific routines described herein may represent one or more of any number of processing strategies such as event-driven, interrupt-driven, multi-tasking, multi-threading, and the like. As such, various actions, operations, and/or functions illustrated may be performed in the sequence illustrated, in parallel, or in some cases omitted. Likewise, the order of processing is not necessarily required to achieve the features and advantages of the example embodiments described herein but is provided for ease of illustration and description. One or more of the illustrated actions, operations and/or functions may be repeatedly performed depending on the particular strategy being used. Further, the described actions, operations and/or functions may graphically represent code to be programmed into non-transitory memory of a computer readable storage medium in a control unit (e.g. a microcontroller) of the system, where the described actions are carried out by executing the instructions in a system including the various hardware components in combination with an electronic control unit.

According to the present invention a method is disclosed, the method characterized in that the method is performed with the system as defined in this disclosure, especially as defined of any of claims 1-9.

In particular, the following aspects are explicitly disclosed according to the present invention:

Aspect 1: A method for providing neuromodulation, especially neurostimulation, comprising the steps of

- providing neuromodulation to the cervical spinal cord of a patient,
- measuring movements and/or muscle activations of a patient,
- implementing a mapping between desired muscle activation patterns and neuromodulation patterns, and
- processing signals indicating movements and/or muscle activations, further forwarding the processed signals and directing the implemented mapping between desired muscle activation patterns and neuromodulation patterns for providing neuromodulation.

Aspect 2: The method according to aspect 1, characterized in that the neuromodulation is a neurostimulation.

Aspect 3: The method according to aspect 1 or aspect 2, characterized in that the neurostimulation is provided in at least three channels independently.

Aspect 4: The method according to one of the preceding aspects, characterized in that the neurostimulation is provided in a frequency range from 0-1000Hz, preferably between 10-1000Hz.

Aspect 5: The method according to one of the preceding aspects, characterized in that the neuromodulation is a neuromodulation for selective muscle activation.

Aspect 6: The method according to the preceding aspects, characterized in that the neuromodulation is configured and arranged to target posterior roots.

**References**

| | |
|---|---|
| 10 | System for neuromodulation/neurostimulation |
| 12 | Neuromodulator/neurostimulator |
| 14 | Dorsal neuromodulation array/ dorsal epidural array |
| 16 | Sensor |
| 18 | Mapping module |
| 20 | Processing module |

| | |
|---|---|
| EX | Electrode X/active site X |
| E1 | Electrode 1/active site 1 |
| E2 | Electrode 2/active site 2 |
| E3 | Electrode 3/active site 3 |
| E5 | Electrode 5/active site 5 |
| E6 | Electrode 6/active site 6 |
| E8 | Electrode 8/active site 8 |

| | |
|---|---|
| APB | Abductor pollicis brevis |
| BIC | Biceps |
| DEL | Deltoid |
| EDC | Extensor digitorum communis |
| FDS | Flexor digitorum superficialis |
| TRI | Triceps |
| WRE | Wrist extensor |
| WRF | Wrist flexor |

**Claims**

1. A system (10) for providing neuromodulation, especially neurostimulation, comprising

   - at least one neuromodulator (12) for neuromodulation of the cervical spinal cord,
   - at least one dorsal neuromodulation array (14) for providing neuromodulation to the cervical spinal cord,
   - at least one sensor (16) for measuring movements and/or muscle activations of a patient,
   - at least one mapping module (18), which is configured and arranged for implementing a mapping between desired muscle activation patterns and neuromodulation parameters, and
   - at least one processing module (20) which is configured and arranged for processing signals provided by the at least one sensor (16), further forwarding the processed signals to the mapping module (18) and directing the mapping module output to the neuromodulator (12).

2. The system (10) according to claim 1, **characterized in that** the neuromodulator (12) is a neurostimulator (12).

3. The system (10) according to claim 1 or claim 2, **characterized in that** the neurostimulator (12) is configured and arranged to provide neurostimulation in at least three channels independently.

4. The system (10) according to one of the preceding claims, **characterized in that** the neurostimulator (12) is configured and arranged to provide neurostimulation in a frequency range from 0-1000Hz, preferably between 10-1000Hz.

5. The system (10) according to one of the preceding claims, **characterized in that** the neuromodulator (12) is at least partially implanted, especially fully implanted.

6. The system (10) according to one of the preceding claims, **characterized in that** the dorsal neuromodulation array (14) is a dorsal neurostimulation array (14).

7. The system (10) according to one of the preceding claims, **characterized in that** the dorsal neuromodulation array (14) has a plurality of active sites E, wherein the active sites E are placed in a region of approx. 2-25 mm, especially approx. 4-10 mm from the midline of the patient's spinal cord.

8. The system (10) according to one of the preceding claims, **characterized in that** the neuromodulation is a neuromodulation for selective muscle activation.

9. The system (10) according to the previous claims, **characterized in that** the mapping stage and the integration stage may be encompassed by the same computer/microcontroller and that one or both are external to the patient, partially implanted or fully implanted.

10. The system (10) according to one of the preceding claims, **characterized in that** the neuromodulation is configured and arranged to target posterior roots.

EP 3 695 879 A1

Fig. 1

Fig. 2

Fig. 3

Fig. 4

Europäisches
Patentamt

European
Patent Office

Office européen
des brevets

# EUROPEAN SEARCH REPORT

Application Number

EP 19 15 6852

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| E | EP 3 495 019 A1 (ECOLE POLYTECHNIQUE FED LAUSANNE EPFL [CH]; GTX MEDICAL B V [NL]) 12 June 2019 (2019-06-12) * paragraphs [0060] - [0062], [0168], [0182] - [0208]; figure 1 * ----- | 1-10 | INV. A61N1/36 |
| A | EP 3 184 145 A1 (ECOLE POLYTECHNIQUE FÉDÉRALE DE LAUSANNE [CH]) 28 June 2017 (2017-06-28) * paragraphs [0043] - [0065], [0148] * ----- | 1-10 | |
| A | WO 2018/033591 A2 (ECOLE POLYTECHNIQUE FED LAUSANNE EPFL [CH]) 22 February 2018 (2018-02-22) * the whole document * ----- | 1-10 | |

TECHNICAL FIELDS
SEARCHED (IPC)

A61N

The present search report has been drawn up for all claims

| Place of search | Date of completion of the search | Examiner |
|---|---|---|
| Munich | 7 August 2019 | Aronsson, Fredrik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 695 879 A1**

## ANNEX TO THE EUROPEAN SEARCH REPORT
## ON EUROPEAN PATENT APPLICATION NO.

EP 19 15 6852

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2019

| Patent document cited in search report | | Publication date | Patent family member(s) | | Publication date |
|---|---|---|---|---|---|
| EP 3495019 | A1 | 12-06-2019 | EP | 3495019 A1 | 12-06-2019 |
| | | | WO | 2019110401 A1 | 13-06-2019 |
| EP 3184145 | A1 | 28-06-2017 | CN | 106902458 A | 30-06-2017 |
| | | | EP | 3184145 A1 | 28-06-2017 |
| | | | US | 2017173326 A1 | 22-06-2017 |
| | | | US | 2017354819 A1 | 14-12-2017 |
| WO 2018033591 | A2 | 22-02-2018 | CN | 109843371 A | 04-06-2019 |
| | | | EP | 3500336 A2 | 26-06-2019 |
| | | | WO | 2018033591 A2 | 22-02-2018 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016030750 A1
- US 2016001096 A1
- EP 2810689 A1
- EP 2810690 A1
- US 2015066111 A1

- EP 2868343 A1
- EP 3269424 A1
- EP 3421081 A1
- EP 3381506 A1

**Non-patent literature cited in the description**

- International perspectives on spinal cord injury. World Health Organization & International Spinal Cord Society. World Health Organization, 2013
- **WENGER N et al.** Spatiotemporal neuromodulation therapies engaging muscle synergies improve motor control after spinal cord injury. *Nat. Med.,* 2016, vol. 22, 138
- **WENGER N.** Closed-loop neuromodulation of spinal sensorimotor circuits controls refined locomotion after complete spinal cord injury. *Sci. Trans/. Med.,* 2014, vol. 6, 255ra133
- **CAPOGROSSO M et al.** A brain-spine interface alleviating gait deficits after spinal cord injury in primates. *Nature,* 2016, vol. 539, 284
- **LU, DC. et al.** Engaging Cervical Spinal Cord Networks to Reenable Volitional Control of Hand Function in Tetraplegic Patients. Neurorehabil. *Neural Repair,* 2016, vol. 30, 951-962
- **SHARPE AN et al.** Upper-limb muscle responses to epidural, subdural and intraspinal stimulation of the cervical spinal cord. *J. Neural Eng.,* 2014, vol. 11, 016005-016005
- **BIZZI E et al.** Modular organization of motor behavior in the frog's spinal cord. *Trends in neurosciences,* 1995, vol. 18, 442-446
- **LEVINE AJ et al.** Identification of a cellular node for motor control pathways. *Nature neuroscience,* 2014, vol. 17, 586-593
- **VAN DEN BRAND R et al.** Restoring Voluntary Control of Locomotion after Paralyzing Spinal Cord Injury. *Science,* 2012, vol. 336, 1182-1185
- **ANGELI CA.** Itering spinal cord excitability enables voluntary movements after chronic complete paralysis in humans. *Brain: a journal of neurology,* 2014, vol. 137, 1394-1409

- **HARKEMA S et al.** Effect of epidural stimulation of the lumbosacral spinal cord on voluntary movement, standing, and assisted stepping after motor complete paraplegia: a case study. *The Lancet,* vol. 377, 1938-1947
- **DANNER SM et al.** Human spinal locomotor control is based on flexibly organized burst generators. *Brain: a journal of neurology,* 2015, vol. 138, 577-588
- **COURTINE G et al.** Transformation of nonfunctional spinal circuits into functional states after the loss of brain input. *Nature neuroscience,* 2009, vol. 12, 1333-1342
- **CAPOGROSSO M et al.** A brain-spine interface alleviating gait deficits after spinal cord injury in primates. *Nature,* 2016, vol. 539, 284-288
- **CAPOGROSSO M et al.** A computational model for epidural electrical stimulation of spinal sensorimotor circuits. *The Journal of neuroscience: the official journal of the Society for Neuroscience,* 2013, vol. 33, 19326-19340
- **MORAUD EM et al.** Mechanisms Underlying the Neuromodulation of Spinal Circuits for Correcting Gait and Balance Deficits after Spinal Cord Injury. *Neuron,* 2016, vol. 89, 814-828
- **RATTAY F et al.** Epidural electrical stimulation of posterior structures of the human lumbosacral cord: 2. quantitative analysis by computer modeling. *Spinal cord,* 2000, vol. 38, 473-489
- **GERASIMENKO Y et al.** *Soc. Neurosci. Abstr.*
- **MINASSIAN K et al.** Human lumbar cord circuitries can be activated by extrinsic tonic input to generate locomotor-like activity. *Human Movement Science,* 2007, vol. 26, 275-295
- **ANGELI CA et al.** Altering spinal cord excitability enables voluntary movements after chronic complete paralysis in humans. *Brain: a journal of neurology,* 2014, vol. 137, 1394-1409

- **HARKEMA S. et al.** Effect of epidural stimulation of the lumbosacral spinal cord on voluntary movement, standing, and assisted stepping after motor complete paraplegia: a case study. *The Lancet,* 1938, vol. 377
- **WENGER N et al.** Spatiotemporal neuromodulation therapies engaging muscle synergies improve motor control after spinal cord injury. *Nat Med,* 2016, vol. 22, 138-145
- **CAPOGROSSO M et al.** A Computational Model for Epidural Electrical Stimulation of Spinal Sensorimotor Circuits. *Journal of Neuroscience,* 04 December 2013, vol. 33 (49), 19326-19340
- **COURTINE G et al.** Transformation of nonfunctional spinal circuits into functional states after the loss of brain input. *Nat Neurosci,* 2009, vol. 12 (10), 1333-1342
- **MORAUD EM et al.** Mechanisms Underlying the Neuromodulation of Spinal Circuits for Correcting Gait and Balance Deficits after Spinal Cord Injury. *Neuron,* 2016, vol. 89 (4), 814-828
- **SCHMIDLIN E.** Behavioral assessment of manual dexterity in non-human primates. *J. Vis. Exp. JoVE,* 2011, vol. 3258
- **CHATAGNY P et al.** *Distinction between hand dominance and hand preference in primates: A behavioral investigation of manual dexterity in nonhuman primates (macaques) and human subjects,* 2013, vol. 3
- **JENNY A et al.** Principles of motor organization of the monkey cervical spinal cord. *J. Neurosci.,* 1983, vol. 3, 567
- **MINEV IR et al.** Electronic dura mater for long-term multimodal neural interfaces. *Science,* 2015, vol. 347, 159
- **SHERMAN JL et al.** Measurements of the normal cervical spinal cord on MR imaging. *Am. J. Neuroradiol.,* 1990, vol. 11, 369-372
- **KIRAZLI Ö et al.** Anatomy of the spinal dorsal root entry zone: its clinical significance. *Acta Neurochir. (Wien),* 2014, vol. 156, 2351-2358